# EUROPÄISCHE PATENTANMELDUNG

(11) **EP 0 717 974 A2**
(43) Veröffentlichungstag der Anmeldung: **26.06.1996**
(21) Anmeldenummer: 95890221.5
(22) Anmeldetag: 18.12.1995
(51) Int. Cl.: A61H 23/04, A61H 39/00

(54) **Fussreflexzonenmassagevorrichtung**

(30) Priorität: 23.12.1994 AT 2401/94
(71) Anmelder: Bumba, Walter, A-1220 Wien (AT)
(72) Erfinder: Bumba, Walter, A-1220 Wien (AT)
(74) Vertreter: Kliment, Peter

(57) **Zusammenfassung**

Vorrichtung zum Massieren und/oder Stimulieren einer Person, mit mehreren auf zumindest einem Tragteil vorzugsweise in Reihen ausgerichteten Massageelementen, von denen jedes einen in einem Betätigungszylinder (8) geführten Massagekolben (10) aufweist, dessen Kolbenlängsachse (10') im wesentlichen etwa normal zur Oberfläche des zu behandelnden Körperteils einer Person angeordnet ist, wobei der Massagekolben (10) entlang seiner Kolbenachse (10') durch eine mit einer Steuereinheit (21) verbundene erste Verschiebeeinrichtung (15) gesteuert hin- und wegbewegbar ist und jedes aus dem Betätigungszylinder (8) herausragendes freies Kolbenende (11) stirnseitig einen Kontaktbereich (12) aufweist, und die Kolbenlängsachsen (10') benachbarter Massageelemente (9) etwa parallel zueinander angeordnet sind. Um mit einer solchen Vorrichtung Fußreflexzonen-Massagen durchführen zu können, ist vorgesehen, daß mehrere Reihen von Massageelementen nebeneinander angeordnet sind und die Steuereinheit (21) mit einer Datenbank (45) verbunden ist, in der die Fußreflexzonen (2; 3) als Funktion der Abmessungen des Fußes (41, 4r) der zu behandelnden Person abgelegt sind.

## Beschreibung

Die Erfindung betrifft eine Vorrichtung zum Massieren und/oder Stimulieren einer Person gemäß dem Oberbegriff des Anspruches 1.

Es sind einfache mechanische Vorrichtungen zum Massieren bzw. Stimulieren der sohlenseitigen Fußreflexzonen bekannt, welche aus einer Anzahl von parallelen zylindrischen Rollenanordnungen bestehen. Derartige Vorrichtungen vermögen allerdings nur eine relative globale Stimulation und vermögen nicht auf spezifische lokale Zonen einzugehen. Dies ist allerdings notwendig, um durch Fußreflexzonenmassage ganz gezielt Beschwerden, Fehlentwicklungen und Krankheiten entgegensteuern und behandeln zu können. Auf diese Weise können die Krankheitsbilder und der Verlauf zahlreicher Erkrankungen und Beschwerden, wie beispielsweise Herz- Lungen- und Kreislauferkrankungen, Tumore, Schulter- und Rückenbeschwerden, Paralyse, Phlebitis, Nieren- und Gallenleiden, Hautkrankheiten, Augenkrankheiten, Erkrankung der Verdauungsorgane, etc. zumindest günstig beeinflußt werden.

Wirksame Fußreflexzonenmassagen konnten daher bisher nur in Form von manueller Kontaktmassagen von speziell ausgebildeten Therapeuten durchgeführt werden. Der Erfolg einer Behandlung hängt dabei stets vom fachlichen Können und auch der Tagesverfassung des Masseurs ab und kann daher sehr unterschiedlich sein.

Im routinemäßigen Spitalsbetrieb, wo mittlerweile auch Fußreflexzonenmassagen in bestimmten Fällen, beispielsweise bei Dekubituspatienten zu den unterstützenden Behandlungsmethoden zählen, werden an einen Fußreflexzonenmasseur hohe physische und psychische Anforderungen gestellt. Seine Behandlung muß wirksam und wiederholbar sein, daß heißt er muß bei jedem Patienten in jeder Behandlung die richtige Reflexzone unter den manchmal sehr dicht benachbarten Fußreflexzonen stimulieren, um den gewünschten Effekt zu erzielen. Dies ist insbesondere unter Zeitdruck nicht immer gewährleistet.

Vorrichtungen der eingangs erwähnten Art wurden z.B. durch die GB2230454 A und die WO 88/08290 bekannt, die beide physiotherapeutische Vorrichtungen zur Behandlung der Wirbelsäule vorschlagen. Dabei sind die Massagekolben im wesentlichen in einer Reihe angeordnet und werden über eine Steuereinrichtung mit einem Druckmedium beaufschlagt. Dabei ist vorgesehen, daß eine eher schlagartige Beanspruchung auftritt, um einen Reflex auszulösen und danach eine Pause eingehalten wird, bevor eine neuerliche Beaufschlagung erfolgt.

Weiters wurde durch die US 2 638 090 A eine Vorrichtung zum Massieren der Füße vorgeschlagen, bei der dicht an dicht liegende aufweitbare Schläuche vorgesehen sind, die über eine Ventilsteuerung abwechseln mit einer Druckquelle und dem Umgebungsdruck verbunden werden.

Eine Stimulierung eng begrenzter Bereich, wie diese bei der Fußreflexzonenmassage erforderlich ist, ist mit diesen bekannten Vorrichtungen nicht möglich.

Aufgabe der Erfindung ist es, diese Nachteile zu vermeiden und eine Vorrichtung zu schaffen, mit der eine individuelle Stimulierung eng begrenzter Bereiche, wie sie für die Fußreflexzonenmassage erforderlich ist, auf einfache Weise erreicht werden kann.

Erfindungsgemäß wird dies bei einer Vorrichtung der eingangs erwähnten Art durch die kennzeichnenden Merkmale des Anspruches 1 erreicht.

Durch Anordnung der Massageelemente in mehreren Reihen können praktisch beliebige Punkte des Fußes eines Patienten stimuliert werden, wobei durch die Steuereinheit für jede Fußgröße die richtige Lage der Fußreflexzonen ermittelt und die Massageelemente punktgenau bewegt werden können.

Durch die Merkmale des Anspruches 2 ist es möglich ganz spezifisch nur einzelne MAssageelemente zu aktivieren und dadurch bestimmte Reflexzonen zu stimulieren.

Durch die Merkmale des Anspruches 3 ergibt sich eine intensive Massagewirkung, wobei die durch die Aktivierungseinrichtung bzw. die elektromagnetischen Erreger erzwungenen Längsschwingungen der MAssageelemente den Stimulationseffekt erhöhen.

Durch die Merkmale des Anspruches 4 ist es möglich, auch in und quer zur Fußlängsrichtung zu massieren, wodurch jede zweidimensionale Massagebewegung, beispielsweise kreisförmige Stimulation, realisierbar ist. In Überlagerung mit der ersten Verschiebeeinrichtung kann jede therapeutische dreidimensionale Massagetechnik am Fuß der zu massierenden Person, spezifisch auf die jeweils angewählten Fußreflexzonen durchgeführt werden.

Die Merkmale des Anspruches 5 ermöglichen es die Vorrichtung auch zur Schwachstrom stimulation der Fußreflexzonen zu verwenden, wobei die Eindringtiefe der Elektroden in die Haut der zu behandelnden Person durch die elektrisch nicht leitenden Auflageflächen der Massageköpfe begrenzt werden können.

Um die gleichen Massageelemente sowohl zur Massage als auch zur Elektrostimulation von Fußreflexzonen oder gleichzeitig zu beiden Behandlungsarten verwenden zu können, ist es vorteilhaft die Merkmale des Anspruches 6 vorzusehen.

Die Merkmale des Anspruches 7 ermöglichen es auf einer Fläche möglichst viele Massageelemente unterzubringen, wobei sich auch eine Vereinfachung der Steuerung der MAssageelemente ergibt, da z.B. dreißig Reihen von Massageelemente steuerungsmäßig zusammengefaßt werden können.

Eine besonders einfache Gestaltung eienr erfindungsgemäßen Vorrichtung ergibt sich durch die Merkmale des Anspruches 8, die sich auch besonders für die Behandlung von bewegungsbehinderten Personen eignet, wobei der zu behandelnde Fuß, ohne die Person übermäßig zu belasten, ehr leicht in die für die Behandlung optimale Position gebracht werden kann. Wenn der Fuß die richtige Lage eingenommen hat, kann der dritte Tragteil von einer Ruhelage in eine Behandlungsposition geschwenkt werden. Außerdem kann durch die jeweilige Verschiebeeinrichtung der gesamte Tragteil in Längsrichtung zum Fuß bzw. quer dazu verschoben werden.

Um die Bestückung der Kolbenenden mit dem passenden Massagekopf oder das Verdrehen des Massagekopfes in die Massage- oder die Elektrostimulationsstellung schon während einer laufenden Behandlung für die nächste Behandlung vorbereiten zu können, um die Stillstandszeiten wegen Umrüstarbeiten minimieren zu können, ist es vorteilhaft die Merkmale des Anspruches 9 vorzusehen.

Die Merkmale des Anspruches 10 ermöglichen eine Ruhigstellung und Abstützung des Fußes der zu behandelnden Person wäherend der Behandlung. Dabei kann das Aufblasen des Elementes mit dem richtigen Druck kann dabei über die Steuereinrichtung erfolgen, welche über einen Drucksensor der Haltevorrichtung den Elementinnendruck mißt und optimal einstellt.

Durch die Merkmale des Anspruches 11 können therapeutisch koordinierten Massage- bzw. Stimulationsabläufe für jede Behandlungsart einmalig durch einen ausgebildeten Therapeuten beispielsweise über einen sensorbestückten Handschuh eingegeben und gespeichert werden. Durch Abrufen der Daten können mittels der durch die Verschiebeeinrichtungen dreidimensional bewegten Massageelemente die Bewegungsabläufe des Therapeuten exakt wiederholt werden.

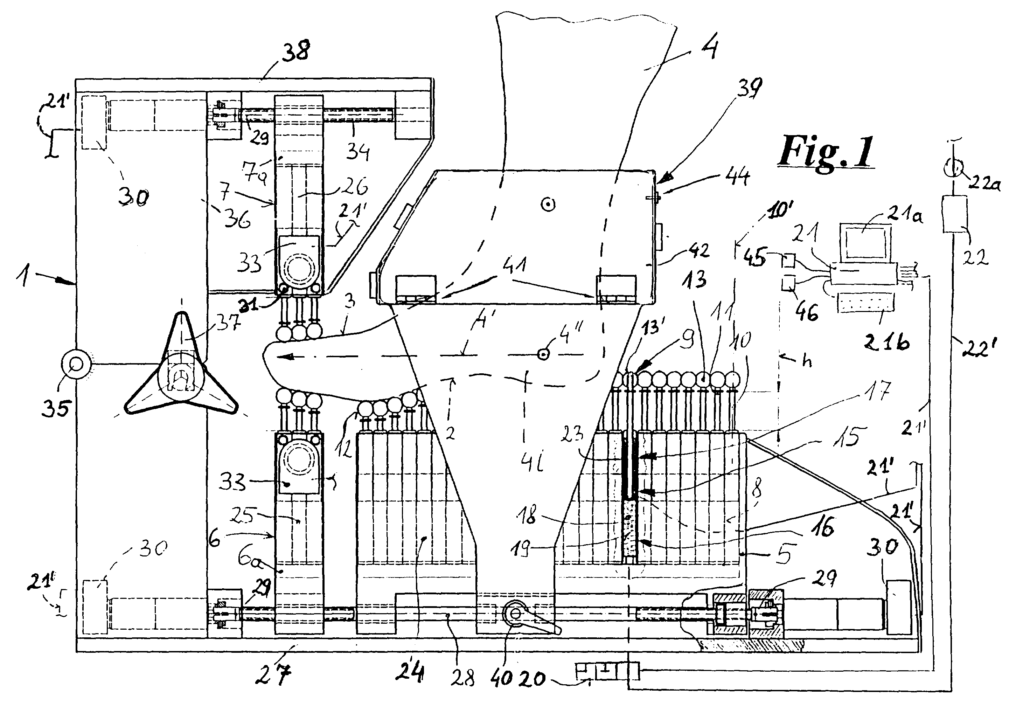

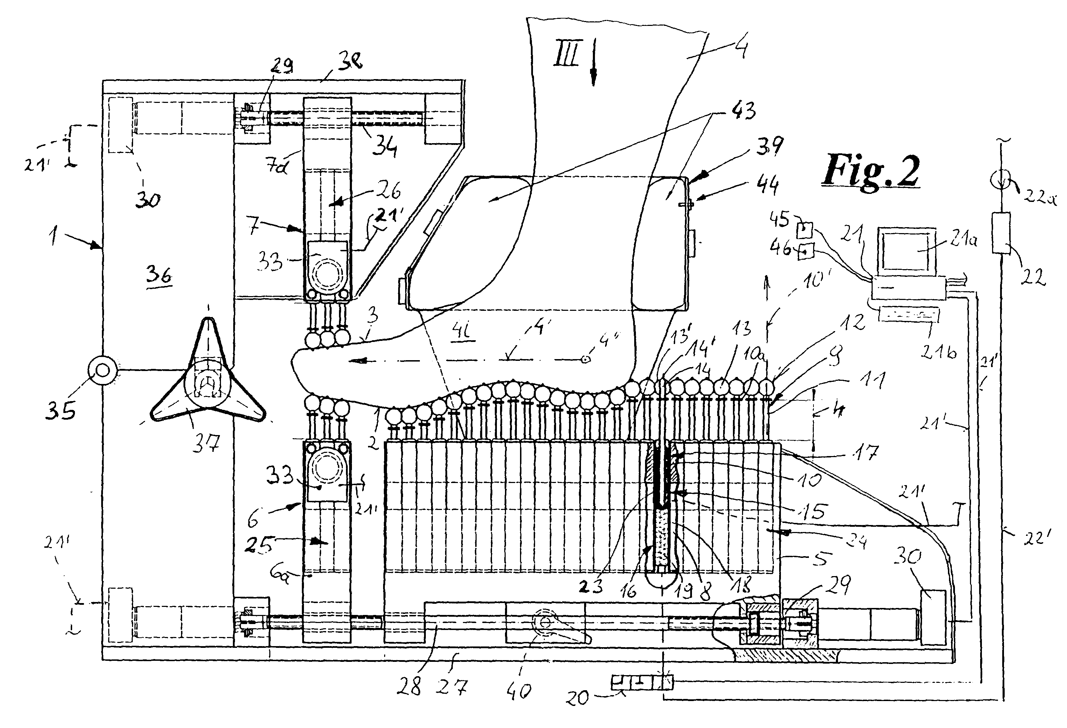

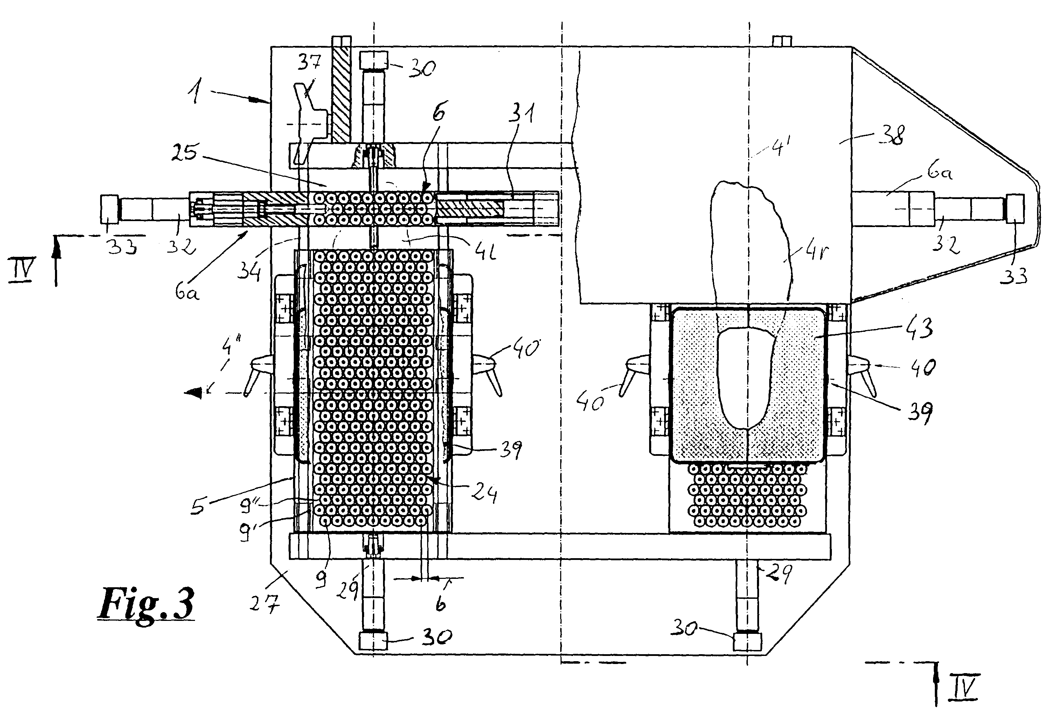

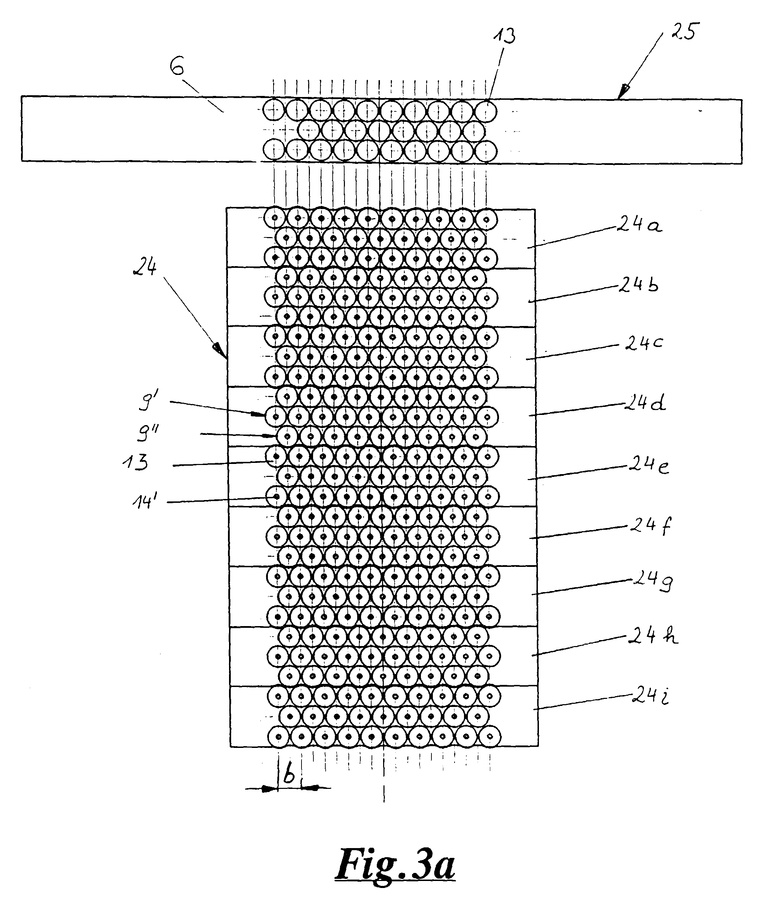

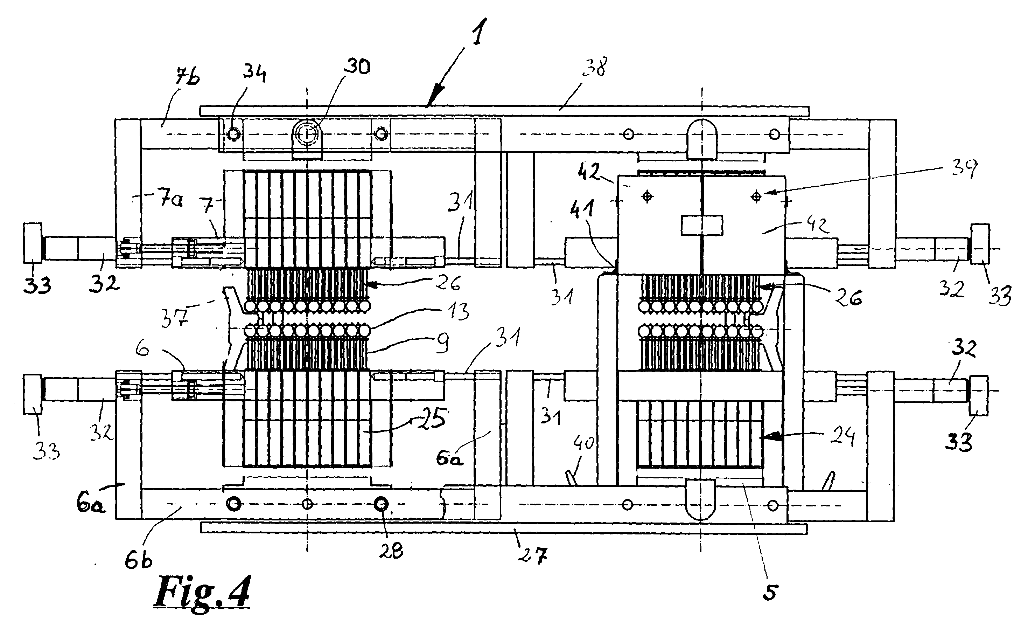

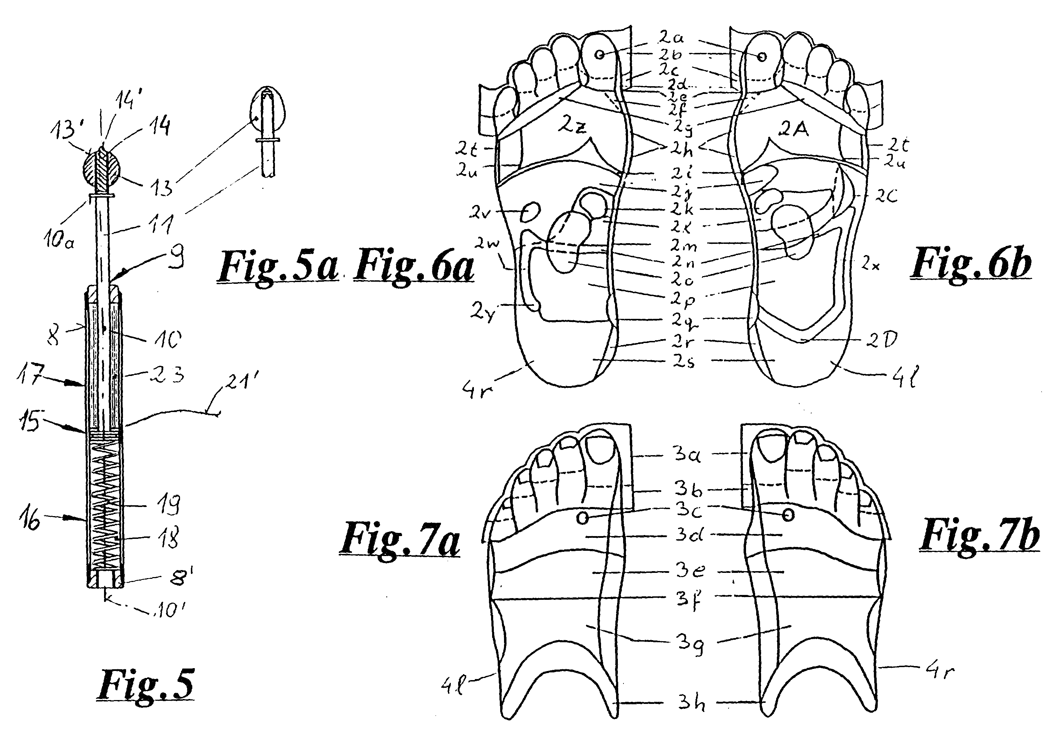

Die Erfindung wird an Hand eines in den Figuren gezeigten Ausführungsbeispieles näher erläutert. Es zeigen Fig.1 bzw. 2 die erfindungsgemäße Vorrichtung mit Massageelementen zur Massage- bzw. Stimulationsbehandlung, Fig.2 die Vorrichtung in einer Seitenansicht, Fig.3 die gleiche Vorrichtung in einer Ansicht aus der Richtung III in Fig.2 mit teilweise weggeschnittenem Gehäuse, Fig. 3a ein Detail der Fig. 3, Fig.4 eine weitere Ansicht der Vorrichtung mit Teilschnitt gemäß der Linie IV-IV Fig.3, Fig.5 ein einzelnes Massageelement im Detail, Fig.5a eine weitere Ausführungsvariante eines Massagekopfes, Fig.6a und Fig.6b schematisch die Lage der sohlenseitigen Fußreflexzonen, Fig.7a und Fig.7b die Lage der ristseitigen Fußreflexzonen.

Funktionsgleiche Teile tragen die gleichen Bezugszeichen.

Fig.1 zeigt die zur Massagebehandlung vorbereitete Vorrichtung 1, die Fig.2 bis 4 zeigen die gleiche Vorrichtung während einer Stimulationsbehandlung. mit teilweise weggeschnittenen bzw. weggeklappter Haltevorrichtung.

Die in den Fig.1 und 2 dargestellte erfindungsgemäße Vorrichtung 1 zum Massieren oder Stimulieren von Fußreflexzonen 2 und 3 einer Person 4 weist für den rechten und linken Fuß 4r und 41 jeweils einen ersten Tragteil 5, einen zweiten Tragteil 6 und einen dritten Tragteil 7 auf. In jedem Tragteil 5, 6 und 7 sind eine Anzahl von parallelen Betätigungszylindern 8 zur Aufnahme von Massageelementen 9 vorgesehen.

Wie in Fig.5 im Detail dargestellt ist, besteht jedes Massageelement 9 aus einem im Betätigungszylinder 8 entlang der Kolbenlängsachse 10' um eine Hubhöhe h bewegbaren Massagekolben 10, dessen aus dem Betätigungszylinder 8 in Richtung der Fußreflexzonen 2 bzw. 3 herausragendes freies Kolbenende 11 stirnseitig einen Kontaktbereich 12 zum Fuß 4r bzw. 41 der Person 4 aufweist. Der Kontaktbereich 12 kann entweder direkt durch das Kolbenende 11 oder durch einen auf dem Kolbenende 11 lösbar befestigten Massagekopf 13 gebildet sein, der beispielsweise kalottenförmig oder ellipsoidförmig gestaltet sein kann, wie in Fig.5a gezeigt ist. In dem in den Fig.2 bis 4 gezeigten Ausführungsbeispiel und der Detaildarstellung in Fig.5 weist das freie Kolbenende 11 an seiner Stirnseite eine Elektrode 14 für galvanische Behandlungen auf. Im Falle der galvanischen Stimulation von Fußreflexzonen 2 und 3 wird aufjedes freie Kolbenende 11 ein eine zentrische Bohrung aufweisender Massagekopf 13 aufgesetzt, der von der Elektrode 14 derart durchdrungen wird, daß gerade die Spitze 14' der Elektrode 14 noch von der Oberfläche des Massagekopfes 13 hervorragt. Die Oberfläche des Massagekopfes 13 bildet somit eine Auflagefläche für den Fuß 4r oder 41 und verhindert ein zu tiefes Eindringen Spitze 14' der Elektrode 14 in die Haut der Person 4. Der am Massagekolben 10 angebrachte Abstandhalter 10' begrenzt den Kolbenhub.

Zur axialen Bewegung jedes Massagekolben 10 ist pro Massageelement 9 eine erste Verschiebeeinrichtung 15 vorgesehen, die eine pneumatisch bzw. hydraulisch betätigbare Aktivierungseinrichtung 16 und einen elektromagnetisch betätigbaren Erreger 17 aufweist.

Jede pneumatisch bzw. hydraulisch betätigbare Aktivierungseinrichtung 16 besteht aus einem mit dem Massagekolben 10 wirkverbundenen Druckraum 18 innerhalb des Betätigungszylinders 8 zur Hebung oder Absenkung eines Massagekolbens 10. Zur Definition einer Nullstellung des Massagekolbens 19 kann im Druckraum 18 eine Feder 19 vorgesehen sein, die in Kolbenlängsachse 10' angeordnet ist. Das eine Federende drückt auf das dem Massagekopf 13 gegenüberliegende Ende des Massagekolbens 10 und das andere Federende stützt sich über eine innere Schulter 8' des Betätigungszylinders 8 ab. Jeder Druckraum 18 ist über jeweils ein Steuerventil 20 mit einer Druckleitung 22' strömungsverbindbar. Die Druckleitung 22' ist mit einem Druckspeicher 22 verbunden welcher beispielsweise von einer Pumpe 22a gespeist wird.

Jeder elektromagnetische Erreger 17 besteht aus einer den zumindest teilweise aus ferromagnetischem Material bestehenden Massagekolben 10 im Betätigungszylinder 8 umgebenden Induktionsspule 23, welche Über die Steuereinheit 21 mit Wechselstrom beaufschlagbar ist. In einer zweiten Funktion dient die Induktionsspule 23 zusammen mit dem ferromagnetischen Kern des Massagekolbens 10 als induktiver Wegaufnehmer, welcher die Daten über die genaue Lage des Massageelements 9 über die Steuerleitung 21' an die Steuereinheit 21 weitergibt.

Die Massageelemente 9 sind gruppenweise zu einer ersten, zweiten und dritten Gruppe 24, 25 und 26 zusammengefaßt. Dabei sind die Massageelemente zweier benachbarter Reihen 9', 9" innerhalb einer Gruppe 24, 25, 26 um jeweils eine halbe Massageelementsbreite b, wie in Abbildung 3a dargestellt versetzt angeordnet.

Die Gruppe 24 jener Massageelemente 9, die sich unter der Fußsohle im Bereich der Ferse bis zum Ballen befinden umfassen etwa 20 bis 30 Reihen und sind zu Untergruppen von vorzugsweise 3 Reihen 24a - 24i zusammengefaßt. Diese Untergruppen 24a - 24 i können als Einheiten aus dem Tragteil 5 entnommen und in dieses wieder eingesteckt werden. Die Gruppe 25 und 26 von Massageelementen 9, die sich unter bzw. über dem Zehenbereich befinden sind vorzugsweise in dreireihigen Einheiten angeordnet und können ebenfalls aus ihren Tragteilen 6 und 7 entnommen bzw. eingesetzt werden. Durch die gruppenweise Zusammenfassung von Massageelementen 9 kann die Vorrichtung 1 ohne lange Stillstandszeiten durch einfaches Wechseln der Untergruppen 24a-24i bzw. der Gruppen 25 und 26 von Massagebehandlung auf Stimulations- bzw. galvanische Behandlung umgerüstet werden.

Der Tragteil 5 für die erste Gruppe 24 von Massageelementen 9, die unter der Fußsohle, im Bereich zwischen Ferse und Ballen angeordnet sind, ist in Richtung der Längsachse 4' des Fußes 4r bzw. 41 auf einem feststehenden Rahmen 27 entlang der Längsführungen 28 verschiebbar gelagert. Durch eine am Tragteil 5 angreifende erste Verschiebeeinrichtung 30 wird der Tragteil 5 in Fußlängsrichtung 4' oszillierend verschoben. Die erste Verschiebeeinrichtung 30 kann ein pneumatisch, hydraulisch oder elektromagnetisch betätigbarer Linearmotor sein. In dem in den Figuren gezeigten Ausführungsbeispiel weist die erste Verschiebeeinrichtung 30 einen Verschiebekolben 29 auf.

Der Tragteil 6 ist in einem U- förmigen Tragrahmen 6a, 6b über Querführungen 31 in Fußquerrichtung 4" verschieblich gelagert. An dem außenliegenden Tragrahmenschenkel 6a ist eine zweite Verschiebeeinrichtung 33 befestigt, die über einen Verschiebekolben 32 oder andere Linearantriebe den Tragteil 6 in Querrichtung 4" des Fußes oszillierend verschiebt. Die Tragrahmenbasis 6b wiederum ist über die Längsführungen 28 in Fußlängsrichtung 4' beweglich gelagert. Der Verschiebekolben 29 einer weiteren ersten Verschiebeeinrichtung 30 , die am Rahmen 27 befestigt ist, versetzt den U-förmigen Tragrahmen 6a, 6b in eine oszillierende Bewegung in Fußlängsrichtung 4'. Das Tragteil 6 kann also unabhängig voneinander in Fußlängsrichtung 4' und Fußquerrichtung 4" Bewegungen ausführen, die von der Steuereinheit 21 kontrolliert werden.

Analog zum Tragteil 6 ist das Tragteil 7 angeordnet. Im U-förmigen Tragrahmen 7a, 7b ist der Tragteil 7 über die Querführung 31 in Fußquerrichtung 4" beweglich gelagert. Eine weitere zweite Verschiebeeinheit 33 bewirkt, daß der Tragteil 7 in Fußquerrichtung 4" eine oszillierende Bewegung ausführt. Die Tragrahmenbasis 7b ist wiederum über die zwei Längsführungen 34 in Längsrichtung 4' des Fußes verschieblich gelagert. Eine weitere erste Verschiebeeinrichtung 30, die am Gehäuse 38 befestigt ist greift mit dem Verschiebekolben 29 an der Tragrahmenbasis 7b an und versetzt den U-förmigen Tragrahmen 7a, 7b zusammen mit den Tragteil 7 in eine in Fußlängsrichtung 4' oszillierende Bewegung. Somit kann auch das Tragteil 7 unabhängig voneinander in Fußlängs- 4' und -Querrichtung 4" Bewegungen ausführen, die von der Steuereinheit 21 kontrolliert werden.

Zum Ver- und Entriegeln des Klapprahmens 36 ist pro linkem und rechten Klapprahmen 7 eine Verschlußschraube 37 vorgesehen. Zum Schutz ist die Vorrichtung 1 noch durch ein Gehäuse 38 umgeben.

Obwohl bei den meisten Behandlungen die Massageelemente 9 der ersten Gruppe 24 nur in Fußlängsrichtung 4' und quer zu den Fußreflexzonen 2 bewegt werden müssen, ist es im Bedarfsfall selbstverständlich auch denkbar, analog zum zweiten und dritten Tragteil 6 und 7 auch den ersten Tragteil 5 mit einer dritten Verschiebeeinrichtung 33 zu versehen um eine Bewegung in Fußquerrichtung 4" zu ermöglichen.

Weiters weist die Vorrichtung 1 eine Halteeinrichtung 39 zum Ruhigstellen des zu massierenden bzw. stimulierenden Fußes 41, 4r auf. Die Halteeinrichtung 38 ist an den Längsführungen 28 verschiebbar mit dem Rahmen 27 verbunden und kann über die Feststellschraube 40 fixiert werden. Die Haltevorrichtung 38 weist einen über die Scharniere 41 wegklappbaren Teil 42 zum Einführen des Fußes 41, 4r auf und beinhaltet ein manschettenförmiges aufblasbares Element 43. Ein Drucksensor 44 ermittelt den Druck im Innenraum des Elementes 43 und gibt die Werte an die Steuereinheit 21 weiter, welche über das Steuerventil 20 den optimalen Druck im Element 43 einstellt.

Die Behandlung mit der erfindungsgemäßen Vorrichtung 1 kann im Prinzip bei einem stehenden, sitzenden oder liegenden Patienten erfolgen. Vorzugsweise erfolgt die Behandlung bei einer liegenden Person 4, wobei die Vorrichtung 1 mit einem Bett, beispielsweise einem Krankenbett, insbesonders zur Behandlung von Dekubituspatienten, abnehmbar oder fest verbunden ist.

Vor der eigentlichen Behandlung sind vorbereitende Maßnahmen durchzuführen. Zunächst muß der Therapeut die Diagnose stellen und die erforderliche Therapie in Form von Bearbeitungsparametern festlegen. Nach gestellter Diagnose kann die zu behandelnde Krankheit oder die zu behandelnde Beschwerde über eine Eingabeeinheit 21b in die Steuereinheit 21 eingegeben werden. Zusätzlich oder an Stelle davon kann mit einem sensorbestücktem Handschuh die Art, Reihenfolge und Richtung der Bewegungen, als auch die aus der Erfahrung des Therapeuten resultierende Größe des aufzubringenden Massage- bzw. Stimulationsdruckes erfaßt und ebenfalls in die Steuereinheit 21 eingegeben werden.

Je nach auszuwählender Behandlungsart, Massage oder galvanische Stimulation können die zu Untergruppen 24a-24i ein- oder mehrreihig zusammengefaßten Massageelemente 9 ausgetauscht werden. Es kann auch erforderlich sein, daß eine oder mehrere Untergruppen 24a-24i aus dem Tragteil 5 herausgenommen werden. Genauso können auch die Gruppe 25 und/oder 26 auf dem Tragteil 6 und 7 ausgetauscht oder weggelassen werden.

Daraufhin werden die Füße des Patienten vermessen was durch ein Abtasten der Füße mit der Vorrichtung 1 erfolgt. Die wegklappbaren Teile 42 der beiden Halteeinrichtungen 39 und der Klapprahmen 36 werden geöffnet und die Füße der Person 4 können in die Vorrichtung 1 eingelegt werden. Daraufhin werden der Klapprahmen 36 und die Halteeinrichtung 39 wieder geschlossen. Die Feststellschrauben 40 der Halteeinrichtung 39 werden gelöst und der Fuß wird so positioniert, daß die den Tragteilen 6 und 7 zugeordneten Massageköpfe 13 gegenüber den Zehenreflexzonen 2a, 2b, 2c, 3a, 3b, 3c, zu liegen kommen. Die Halteeinrichtungen 39 werden nun mit den Feststellschrauben 40 fixiert und es erfolgt ein Druckbeaufschlagen des aufblasbaren Elements 43 womit die auch Füße im Knöchelbereich fixiert werden. Durch die der Fußoberfläche gegenüberliegenden Auflageflächen 13' der Massageköpfe 13 werden die Massagekolben 10 von einer definierten Nullstellung in eine Abdruckstellung bewegt. Die Fußoberfläche erzeugt also eine Abdruck auf die von den Auflageflächen 13' gebildete Ebene. Die Lage aller Massagekolben 10 wird über die als Lagefühler arbeitenden elektromagnetischen Erreger 17 an der Steuereinheit 21 übertragen, die ihrerseits auf Grund der in einer Datenbank 45 abgelegten Fußreflexzonentafel die statistische Lage der Fußreflexzonen 2 und 3 bei der zu behandelnden Person 4 errechnet und in die Datenbank 46 ablegt.

Falls die Person 4 sich einer wiederholten Behandlung unterzieht, kann auf in früheren Behandlungen gespeicherte Werte zurückgegriffen werden.

Als letzter vorbereitender Schritt werden über die Steuereinheit 21 jene Aktivierungseinrichtungen 16 angesteuert, die für die festgelegte Behandlung ermittelt wurden. Dabei werden die Massagekolben 10 von ihrer Abdruckstellung in eine Arbeitsstellung bewegt und es wird ein von der Steuereinheit kontrollierter Druck von den Massageköpfen 13 auf die Fußflächen ausgeübt.

Damit sind die vorbereitenden Maßnahmen abgeschlossen und es kann mit der eigentlichen Massage bzw. Stimulierung begonnen werden. Die Steuereinheit 21 kann nun alle zur Behandlung nötigen Bearbeitungsparameter aus der Datenbank 46 abrufen und betätigt auf Grund dieser Informationen entsprechend die ersten, zweiten und dritten Verschiebeeinrichtungen 15, 30 und 33. Zusätzlich können jene Massagekolben 10, die sich in Arbeitsstellung befinden auch unter elektrische Spannung gesetzt werden um eine galvanische Stimulierung der entsprechenden Fußzonen zu bewirken.

In Fig.6a und Fig.6b ist die sohlenseitige Lage und in den Fig.7b und 7a die ristseitige Lage der Fußreflexzonen 2 und 3 für den rechten bzw. linken Fuß 4r bzw. 41 dargestellt. Die sohlenseitigen Fußreflexzonen 2 sind durch folgende Bezugszeichen markiert: Hypophyse 2a, Kopf-Nebenhöhlen 2b, Hals-Schilddrüse 2c, Nebenschilddrüse 2d, siebenter Halswirbel 2e, Thymusdrüse 2f, Auge/Ohr 2g, Wirbelsäule 2h, Zwerchfell-Sonnengeflecht 2i, Leber 2j, Nebennierendrüsen 2k, Bauchspeicheldrüse 2l, Gürtellinie 2m, Querliegender Dickdarm 2n, Niere 2o, Dünndarm 2p, Blase 2q, Steißbein 2r, Kreuzbein 2s, Arm 2t, Schulter 2u, Gallenblase 2v, aufsteigender Dickdarm 2w, absteigender Dickdarm 2x, Bauhinsche Klappe 2y, Lunge 2z, Lunge/Herz 2A, Magen 2B, Milz 2C, Sigmoid-Dickdarm 2D.

Die ristseitigen Fußreflexzonen tragen die folgenden Bezugszeichen: Kopf/Nebenhöhlen 3a, Hals-Schilddrüse 3b, Lymphkanal 3c, Brust/Lunge Schultergürtel 3d, mittlerer Wirbelsäulenbereich 3e, Gürtellinie 3f, Kreuzbein/Beckenraum 3g, Lymphwege/Leisten Eileiter 3h.

Die in den Fig.6a, 6b und 7a, 7b nur exemplarisch dargestellten Fußreflexzonen sind in der Datenbank 45 abgelegt und können von der Steuereinheit 21 abgefragt werden. Weiters werden in der Datenbank 46 die für jede durch Fußreflexzonenmassage bzw. -stimulation behandelbare Erkrankung notwendigen therapeutischen Bearbeitungen gespeichert.

Beispielsweise müssen bei Augenbeschwerden sohlenseitige Fußreflexzonen, und zwar das Augen/Ohr-Gebiet 2g am Zehengrund gründlich bearbeitet werden, wobei auch das Hals-Gebiet 2c ein hilfreicher Bezugsbereich ist. Zusätzlich dienen die Nieren-Bereiche 2p als Unterstützung.

Bei Fieber ist das Hypophysengebiet 2a an der Unterseite der großen Zehe in regelmäßigen Abständen durchzuarbeiten, bis die Temperatur fällt.

Andere Behandlungen erfordern die Bearbeitung sowohl von sohlenseitigen Fußreflexzonen 2, als auch von ristseitigen Fußreflexzonen 3. So empfiehlt sich beispielsweise bei Rückenbeschwerden im Nackenbereich die Behandlung sowohl der Unter- als auch der Oberseite sämtlicher Zehen, mit besonderer Berücksichtigung der großen Zehe, insbesondere der kreisförmigen Linie des siebenten Halswirbels 2e um den Zehengrund. Weiters sollen auch die Bereiche von Schultern 2u und Sonnengeflecht 2i einbezogen werden.

## Patentansprüche

1. Vorrichtung zum Massieren und/oder Stimulieren einer Person, mit mehreren auf zumindest einem Tragteil vorzugsweise in Reihen ausgerichteten Massageelementen, von denen jedes einen in einem Betätigungszylinder (8) geführten Massagekolben (10) aufweist, dessen Kolbenlängsachse (10') im wesentlichen etwa normal zur Oberfläche des zu behandelnden Körperteils einer Person angeordnet ist, wobei der Massagekolben (10) entlang seiner Kolbenachse (10') durch eine mit einer Steuereinheit (21) verbundene erste Verschiebeeinrichtung (15) gesteuert hin- und wegbewegbar ist und jedes aus dem Betätigungszylinder (8) herausragendes freies Kolbenende (11) stirnseitig einen Kontaktbereich (12) aufweist, und die Kolbenlängsachsen (10') benachbarter Massageelemente (9) etwa parallel zueinander angeordnet sind, **dadurch gekennzeichnet**, daß mehrere Reihen von Massageelementen nebeneinander angeordnet sind und die Steuereinheit (21) mit einer Datenbank (45) verbunden ist, in der die Fußreflexzonen (2; 3) als Funktion der Abmessungen des Fußes (41, 4r) der zu behandelnden Person abgelegt sind.

2. Vorrichtung nach Anspruch 1, **dadurch gekennzeichnet,** daß die erste Verschiebeeinrichtung (15) eine pneumatisch bzw. hydraulisch betätigbare Aktivierungseinrichtung (16) aufweist, die pro Massageelement (9) einen mit dem Massagekolben (10) wirkverbundenen Druckraum (18) im Betätigungszylinder (8) mit pneumatischem bzw. hydraulischem Druck beaufschlägt, wobei der Massagekolben (10) über eine vorzugsweise mit diesem verbundene Feder (16) eine definierte Nullstellung aufweist und über die Aktivierungseinrichtung in eine individuelle Arbeitsstellung bringbar ist.

3. Vorrichtung nach Anspruch 1 oder 2, **dadurch gekennzeichnet**, daß die erste Verschiebeeinrichtung (15) pro Massageelement (9) einen elektromagnetisch betätigbaren Erreger (17) aufweist, wobei vorzugsweise der zumindest teilweise aus ferromagnetischem Material bestehende Massagekolben (10) von einer feststehenden Induktionsspule (23) im Betätigungszylinder (8) umgeben ist und der Massagekolben (10) durch den elektromagnetischen Erreger (17) in eine erste oszillierende Bewegung parallel zu der Kolbenlängsachse (10') versetzbar ist.

4. Vorrichtung nach einem der Ansprüche 1 bis 3, **dadurch gekennzeichnet**, daß mindestens eine Reihe (9', 9") von Massageelementen (9) durch zumindest eine mit der Steuereinheit (21) verbundene zweite und eine dritte Verschiebeeinrichtung (30, 33) in weitere oszillierende Bewegungen etwa in Fußlängsrichtung (4') und Fußquerrichtung (4") versetzbar ist.

5. Vorrichtung nach einem der Ansprüche 1 bis 4, **dadurch gekennzeichnet**, daß auf dem freien Kolbenende (11) ein kalottenförmig oder ellipsoidförmig ausgebildeter Massagekopf (13) lösbar befestigbar, vorzugsweise aufschraubbar, ist und der Kontaktbereich des freien Kolbenendes (11) eine Elektrode (14), vorzugsweise mit einer punktförmigen Kontaktstelle (14') aufweist und die Elektrode (14) von einer, vorzugsweise kalottenförmigen, elektrisch nichtleitenden Auflagefläche (13') eines Massagekopfes (13) umgeben ist.

6. Vorrichtung nach Anspruch 5, **dadurch gekennzeichnet,** daß der Massagekopf (13) in zumindest zwei Stellungen am Kolbenende (11) befestigbar ist, wobei die Elektrode (14) gegenüber den Fußreflexzonen (2, 3) in der ersten Stellung durch den Massagekopf (13) zumindest teilweise abgedeckt und in der zweiten Stellung freigegeben ist, wobei die beiden Stellungen durch zwei Schraubpositionen des Massagekopfes (13) auf dem Kolbenende (11) mit unterschiedlichen Überdeckungen definiert sind.

7. Vorrichtung nach einem der Ansprüche 1 bis 6, **dadurch gekennzeichnet,** daß zwei benachbarte Reihen (9', 9") von Massageelementen (9) jeweils um eine halbe Massageelementbreite (b) gegeneinander verschoben sind, wobei vorzugsweise auf eine Reihe (9') mit gerader Anzahl, vorzugsweise 10, eine Reihe (9") mit ungerader Anzahl, vorzugsweise 9, an Massageelementen (9) folgt, wobei eine erste Gruppe (24) von, vorzugsweise etwa zwanzig bis dreißig, Reihen (9', 9") von die sohlenseitigen Fußreflexzonen (2) vom Fersen- bis zum Ballenbereich eines Fußes (41, 4r) abdeckenden Massageelementen (9) auf einem ersten Tragteil (5) angeordnet ist.

8. Vorrichtung nach einem der Ansprüche 1 bis 7, **dadurch gekennzeichnet**, daß eine zweite Gruppe (25) von, vorzugsweise etwa drei, Reihen (9', 9") von die sohlenseitigen Fußreflexzonen (2) im Zehenbereich eines Fußes (41, 4r) abdeckenden Massageelementen (9) auf einem zweiten Tragteil (6) angeordnet ist und eine dritte Gruppe (26) von, vorzugsweise mindestens drei, Reihen (9', 9") von die ristseitigen Fußreflexzonen (3) zumindest im Zehenbereich eines Fußes (41, 4r) abdeckenden Massageelementen (9) auf einem dritten, wegschwenkbar gelagerten Tragteil (7) angeordnet ist, und die Massageelemente (9) der dritten Gruppe (26) in entgegengesetzter Richtung zu den Massageelementen (9) der ersten und zweiten Gruppe (24, 25) angeordnet sind und an jedem Tragteil (5, 6, 7) eine vorzugsweise einzeln ansteuerbare zweite und/oder dritte Verschiebeeinrichtung (30, 33) angreift.

9. Vorrichtung nach einem der Ansprüche 7 und 8, **dadurch gekennzeichnet**, daß innerhalb zumindest einer Gruppe (24, 25, 26) mehrere Massageelemente (9)in mindestens einer Reihe zu Untergruppen zusammengefaßt sind, und jede Untergruppe von Massageelementen einzeln von den jeweiligen Tragteilen (5, 6, 7) entfernbar bzw. aufsteckbar ist.

10. Vorrichtung nach einem der Ansprüche 1 bis 9, **dadurch gekennzeichnet,** daß zur Ruhigstellung des Fußes (41, 4r) der Person (4) und als Widerlager zu den Massageelementen (9) eine zumindest einen Unterschenkel bzw. Fuß (41, 4r) der Person (4) etwa im Fußgelenksbereich umfassende fixierbare Halteeinrichtung (39) vorgesehen ist, welche vorzugsweise im Kontaktbereich mit dem Unterschenkel bzw. dem Fuß (41, 4r) ein gesteuert aufblasbares Element (43) aufweist.

11. Vorrichtung nach einem der Ansprüche 1 bis 10 **dadurch gekennzeichnet,** daß die Steuereinheit (21) mit einer weiteren Datenbank (46) verbunden ist, in der die therapeutisch koordinierten Massage- bzw. Stimulationsabläufe als Funktion von der jeweiligen Massage- bzw. Stimulationsbehandlung abgelegt sind.
